# EUROPÄISCHE PATENTSCHRIFT

(11) **EP 3 501 309 B1**
(45) Veröffentlichungstag und Bekanntmachung des Hinweises auf die Patenterteilung: **11.05.2022**
(21) Anmeldenummer: 18210092.5
(22) Anmeldetag: 04.12.2018
(51) Int. Cl.: A24F 40/46, A24F 40/70, A24F 40/10

(54) **VERDAMPFERVORRICHTUNG FÜR EINEN INHALATOR, INSBESONDERE FÜR EIN ELEKTRONISCHES ZIGARETTENPRODUKT, UND FERTIGUNGSVERFAHREN**
EVAPORATOR DEVICE FOR AN INHALER, IN PARTICULAR FOR AN ELECTRONIC CIGARETTE PRODUCT, AND PRODUCTION METHOD
DISPOSITIF ÉVAPORATEUR POUR UN INHALATEUR, EN PARTICULIER POUR UN PRODUIT DE CIGARETTE ÉLECTRONIQUE, ET PROCÉDÉ DE FABRICATION

(30) Priorität: 19.12.2017 DE 102017130501
(43) Veröffentlichungstag der Anmeldung: 26.06.2019
(73) Patentinhaber: Hauni Maschinenbau GmbH, 21033 Hamburg (DE)
(72) Erfinder: Rath, Sonali, 79106 Freiburg (DE); Ghanam, Muhannad, 79114 Freiburg (DE); Jaklin, Jan, 73054 Eislingen (DE); Pelz, Uwe, 79227 Schallstadt (DE); Woias, Peter, 79100 Freiburg (DE); Goldschmidtböing, Frank, 77799 Ortenberg (DE)
(74) Vertreter: Müller Verweyen

(56) Entgegenhaltungen:
- EP-A1- 2 316 286
- EP-A1- 3 075 273
- US-A1- 2016 007 653
- US-A1- 2016 345 629

## Beschreibung

Die vorliegende Erfindung betrifft eine Verdampfervorrichtung für einen Inhalator, insbesondere für ein elektronisches Zigarettenprodukt, umfassend einen elektrischen Verdampfer mit einem oder mehreren Heizelementen zum Verdampfen von dem Verdampfer zugeführter Flüssigkeit. Die Erfindung betrifft weiter einen entsprechenden Inhalator, eine Verdampfereinheit und eine Kartusche für einen Inhalator sowie ein Verfahren zur Fertigung einer Verdampfervorrichtung.

In aktuellen elektronischen Zigaretten kommen derzeit zwischen einem und bis zu fünf Verdampferköpfe auf Basis des Docht-Wendels-Prinzips zum Einsatz. Neben den weitverbreiteten Docht-Wendel-E-Zigaretten existieren als weitere Ansätze die flächige Verdampfung von Liquid direkt an der Liquid-Oberfläche, siehe Beispielsweise die Patentanmeldung DE 10 2016 120 803.5. Im Falle der Erhitzung über ein Drahtgitter, auch Mesh genannt, kann sich ein über die Verdampferfläche inhomogenes Temperaturfeld ausbilden, da die Heizleistung über die Gitterfläche zwar im Wesentlichen konstant, der Wärmeabtransport aufgrund lokal unterschiedlicher Liquidnachförderung und gegebenenfalls sogar lokaler Austrocknung jedoch stark inhomogen ist.

Eine Regelung der Verdampfertemperatur über den temperaturabhängigen Heizerwiderstand ist möglich. Da allerdings auf diese Weise nur eine über die gesamte Heizerfläche gemittelte Temperatur erfasst wird, können nur sehr große lokale Temperaturerhöhungen aufgrund von Austrocknung detektiert werden. Zudem würde eine daraufhin erfolgende Reduktion der Heizleistung auch richtig temperierte Bereiche betreffen und dort die Temperatur reduzieren, sodass die erzeugte Dampfmenge reduziert wird.

Eine Aufteilung der Verdampferfläche in einzelne, separat geregelte Bereiche, wie in der Patentanmeldung DE 10 2017 111 119.0 beschrieben, reduziert diese Probleme. Eine mögliche Realisierung eines flächigen Verdampfers mit separierbaren Bereichen ist in der Patentanmeldung DE 10 2016 120 803.5 beschrieben. Die dort beschriebene Silizium-MEMS-Technologie ist jedoch vergleichsweise teuer. Zudem müssen die einzelnen Kanäle des Verdampferchips mit der entsprechenden Regelelektronik elektrisch verbunden werden, was zu einem erhöhten Montageaufwand führt.

Aus der US 2016/345629 A1 ist ein Aerosol generierendes System bekannt, welches einen Heizer mit einer Mehrzahl von Heizfilamenten mit integral ausgeführten elektrischen Kontakten aufweist, wobei die Heizfilamente und die elektrischen Kontakte aus einer Edelstahlfolie gebildet sind.

Eine Inhalatorkomponente für die Bildung eines Kondensationsaerosols durch Verdampfung eines flüssigen Materials mit einem elektrischen Heizelement ist in der EP 3075273 A1 beschrieben. Weitere Heizelemente werden beispielsweise in der EP 2316286 A1 und der US 2016/0007653 A1 beschrieben.

Die Aufgabe der Erfindung besteht darin, eine Verdampfervorrichtung und ein Fertigungsverfahren bereitzustellen, die eine möglichst kostengünstige Fertigung bei gleichzeitig hoher Aerosol- bzw. Dampfqualität und -intensität erlaubt.

Die Erfindung löst diese Aufgabe mit den Merkmalen der unabhängigen Ansprüche.

Die Erfindung schlägt vor, dass ein Träger aus einem Schichtsystem mit einer Polymerfolie und mindestens einer die Polymerfolie flächig kontaktierenden Metallfolie gebildet ist, die Polymerfolie wenigstens eine fluiddurchlässige erste Öffnung aufweist, die Metallfolie wenigstens eine mit der ersten Öffnung kommunizierende fluiddurchlässige zweite Öffnung aufweist, und die Metallfolie die Heizelemente ausbildet, wobei die Heizelemente die zweite Öffnung begrenzend angeordnet sind.

Die Erfindung hat erkannt, dass ein flächiges Verdampfen durch einen folienbasierten Heizer mit einem Schichtaufbau erzielt werden kann, was eine hochqualitative Verdampfung beziehungsweise Aerosolbildung und zugleich eine kostengünstigen Herstellung des Heizers ermöglicht. Der aus dem Schichtsystem durch wenigstens die Polymerfolie und die Metallfolie gebildeten Träger ist folienbasiert und die Heizelemente sind direkt auf dem Träger, welcher auch als Schaltungsträger bezeichnet werden kann, aufgebracht. Die flächige Kontaktierung zwischen der Polymerfolie und der Metallfolie bewirkt einen Verbund, der zum einem elektrisch leitende Bereiche, wie auch elektrisch isolierende Bereiche aufweist, um ein geeignetes Layout des Trägers und somit ein geeignetes Verbinden elektrischer und/oder elektronischer Komponenten zu erlauben. Erfindungsgemäß ist vorgesehen, dass die erste Öffnung der Polymerfolie und die zweite Öffnung kommunizieren, um einen Fluidtransport durch den Träger hinweg zu erlauben. Daher ist vorzugsweise die erste Öffnung in Bereichen angeordnet, welche sich der zweiten Öffnung zuordnen lassen. Die Heizelemente werden durch die zweite Öffnung begrenzende Bereiche der Metallfolie ausgebildet, um eine Verdampfung bzw. Aerosolbildung der durch den Träger strömenden und dem Verdampfer zugeführten Flüssigkeit zu ermöglichen

Vorzugsweise weist die Metallfolie wenigstens an den die Polymerfolie nicht kontaktierenden Abschnitten und/oder den Heizelementen eine passivierende Beschichtung auf, um das Auslösen von Bestandteilen der Metallfolie zu verhindern. Vorzugsweise besteht die passivierende Beschichtung beziehungsweise Passivierung aus einem nichtmetallischen und temperaturstabilen Material, beispielsweise Siliziumdioxid, Quarzglas, oder Siliziumnitrit, welche beispielsweise durch Niedertemperaturabscheidung aufbringbar sind. In anderen Ausführungsformen kommen auch temperaturstabile Polymere, wie Parylen, oder galvanische Beschichtung der Heizelemente mit einem edlen Metall, wie zum Beispiel Gold, infrage.

Erfindungsgemäß umfasst der Träger eine Metalllage, welche die Polymerfolie auf der der Metallfolie gegenüberliegenden Seite kontaktiert, um beispielsweise die Heizelemente und elektrische Leiterbahnen aus unterschiedlichen Materialien fertigen zu können. Die Metalllage ist vorzugsweise mit einer passivierenden Beschichtung versehen, um die Auslösung von Bestandteilen der Metalllage zu verhindern.

Vorzugsweise weist der Träger wenigstens einen Kontakt zum elektrischen Kontaktieren eines elektronischen Bauteils auf, um elektronische Komponenten wie zum Beispiel eine integrierte Steuer-/Regeleinrichtung anbinden zu können. Der Kontakt kann beispielsweise als ein Lötkontakt oder als Steckkontakt ausgeführt sein. Vorzugsweise erstrecken sich die Kontakte durch mehrere Schichten beziehungsweise Folien des Trägers. Beispielsweise können die Kontakte wesentlich durch die Polymerfolie mechanisch gehalten sein, kontaktieren aber die Metallfolie und vorzugsweise die Metalllage, um eine elektrische Verbindung zwischen den leitenden Schichten des Trägers und den in/an den Kontakten angebrachten elektrischen/elektronischen Komponenten zu gewährleisten.

Vorzugsweise weist der Träger Bereiche unterschiedlicher Dicken auf, um den baulichen mechanischen und elektrischen Gegebenheiten Rechnung zu tragen und eine flexible und vielseitige Auslegung der Verdampfervorrichtung zu ermöglichen.

Bevorzugt weist der Träger Bereiche unterschiedlicher Biegesteifigkeit auf, um den Träger in eine Vielzahl von Inhalatoren einbinden zu können. Bevorzugt ist der Träger als ein Starr-Flex-Substrat ausgeführt, welcher miteinander elektrisch und/oder mechanisch verbundene flexible und starre Bereiche aufweist. In anderen Ausführungsformen ist der folienbasierte Träger gänzlich starr oder flexibel.

In einer bevorzugten Ausführungsform weist der Träger eine Flüssigkeitsschnittstelle für die standardisierte Verbindung mit einem Flüssigkeitstank auf, wobei die Flüssigkeitsschnittstelle mit der ersten Öffnung und/oder der zweiten Öffnung kommuniziert, um eine direkte Anbindung der Verdampfervorrichtung an den Flüssigkeitstank oder möglicherweise eine Vielzahl von auswechselbaren Flüssigkeitstanks zu ermöglichen. Bevorzugt weist der Träger mindestens eine Durchgangsöffnung zum Transport von Flüssigkeit von der Flüssigkeitsschnittstelle zu dem Verdampfer auf, um den Transport von Fluid aus den Flüssigkeitstank zum Verdampfer und/oder von verdampftem Liquid vom Verdampfer in einen Luftstrom hinein zu ermöglichen. Die Durchgangsöffnung wird bevorzugt durch die ersten Öffnungen und die zweiten Öffnungen gebildet.

In einer bevorzugten Ausführungsform ist an einer Kante des Trägers ein elektrisches Steckverbinderteil ausgebildet, das zum reversiblen Zusammenwirken mit einem entsprechenden Steckverbinderteil eines Basisteils des Inhalators und zur Versorgung der Verdampfervorrichtung mit elektrischer Energie und/oder zum Empfangen von Steuersignalen für die Verdampfervorrichtung eingerichtet ist, um die Verdampfervorrichtung kostengünstig in einen Inhalator integrieren zu können.

In einer bevorzugten Ausführungsform ist eine elektronische Steuereinrichtung zum Steuern und/oder Regeln der Verdampfervorrichtung auf dem Träger gehalten, um elektronische Komponenten wie zum Beispiel eine integrierte Steuer-/Regeleinrichtung aufnehmen zu können.

Die Erfindung betrifft ferner eine Verdampfereinheit für einen Inhalator umfassend eine Verdampfervorrichtung und ein kapillares Element zur Förderung von Fluid zu dem Verdampfer.

In einer bevorzugten Ausführungsform erstreckt sich das kapillare Element wenigstens teilweise durch die erste Öffnung und/oder die zweite Öffnung, um eine zuverlässige und orientierungsunabhängige Förderung von Liquid beispielsweise aus dem Flüssigkeitstank zum Verdampfer zu ermöglichen.

Die Erfindung betrifft ferner eine Kartusche für einen Inhalator umfassend wenigstens die Verdampfervorrichtung und den Flüssigkeitstank, um die erfindungsgemäße Verdampfervorrichtung in ein standardisiertes Bauteil integrieren zu können. Bevorzugt kommuniziert der Träger, beziehungsweise dessen erste Öffnung und zweite Öffnung, mit dem Flüssigkeitstank, um den Fluidtransport zum Verdampfer zu gewährleisten. Weiter bevorzugt ist die Verdampfervorrichtung, beispielsweise am Träger, auf den Flüssigkeitstank aufgebracht, um einen direkten Kontakt herzustellen. Bevorzugt wird der Kontakt durch eine Flüssigkeitsschnittstelle und/oder ein kapillares Element zur Fluidförderung vermittelt.

Ferner betrifft die Erfindung ein Verfahren zur Fertigung einer erfindungsgemäßen Verdampfervorrichtung, welche durch folgende Schritte aufgebaut wird:
- die Polymerfolie und die Metallfolie werden flächig miteinander verbunden,
- die Metallfolie wird strukturiert, um die zweite Öffnung, Leiterbahnen und/oder Heizelemente auszubilden, und
- die Polymerfolie wird strukturiert, um die erste Öffnung auszubilden. Die genannten Fertigungsschritte können in beliebiger Reihenfolge ausgeführt werden. Im Besonderen können bereits strukturierte Folien flächig auf einander gebracht werden.

Vorzugsweise wird die Metalllage auf der der Metallfolie gegenüberliegenden Seite der Polymerfolie mit der Polymerfolie verbunden. In bevorzugten Ausführungsformen wird bzw. werden die Metallfolie und/oder die Metalllage mit einer passivierenden Beschichtung versehen.

Die Erfindung wird im Folgenden anhand bevorzugter Ausführungsformen unter Bezugnahme auf die beigefügten Figuren erläutert.

### Dabei zeigt

- Fig. 1: eine schematische Darstellung von Komponenten einer E-Zigarette in einer Ausführungsform der Erfindung;
- Fign. 2 und 3: schematische Darstellungen eines Trägers im Schichtaufbau in Ausführungsformen der Erfindung; und
- Fig. 4: eine schematische Darstellung eines Trägers im Schichtaufbau mit Kontakten in einer Ausführungsform der Erfindung.

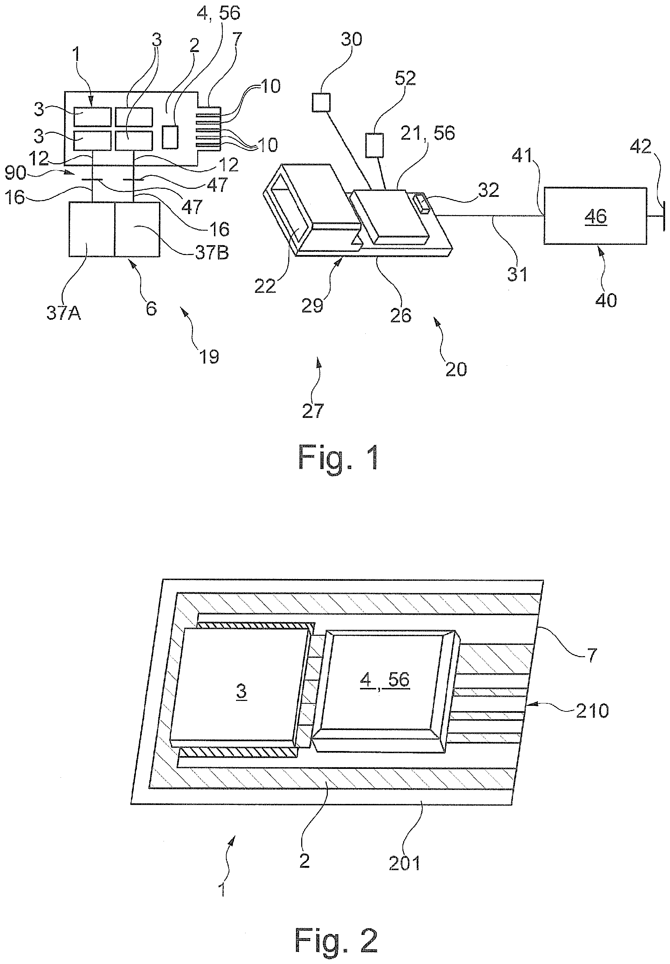

Der in Figur 1 gezeigte Inhalator 27 umfasst eine Verdampfervorrichtung 1 zum Verdampfen von einer oder mehrerer Flüssigkeiten aus einem oder mehreren Flüssigkeitstanks 6 in einer Kartusche 19, und ein vorteilhaft wiederverwendbares Basisteil 20.

Die Verdampfervorrichtung 1 umfasst vorteilhaft einen folienbasierten Träger 2, der mit einem Flüssigkeitstank 6 verbunden oder verbindbar ist. Die Verdampfervorrichtung 1 weist einen Verdampfer 3 oder mehrere Verdampfer 3 auf, die zum Verdampfen von dem oder den Verdampfern 3 aus dem Flüssigkeitstank 6 zugeführter Flüssigkeit dienen. In der Ausführungsform nach Figur 1 sind die Verdampfer 3 beispielsweise in Matrixform, hier beispielsweise vier Verdampfer 3 in 2x2 Matrixform, angeordnet.

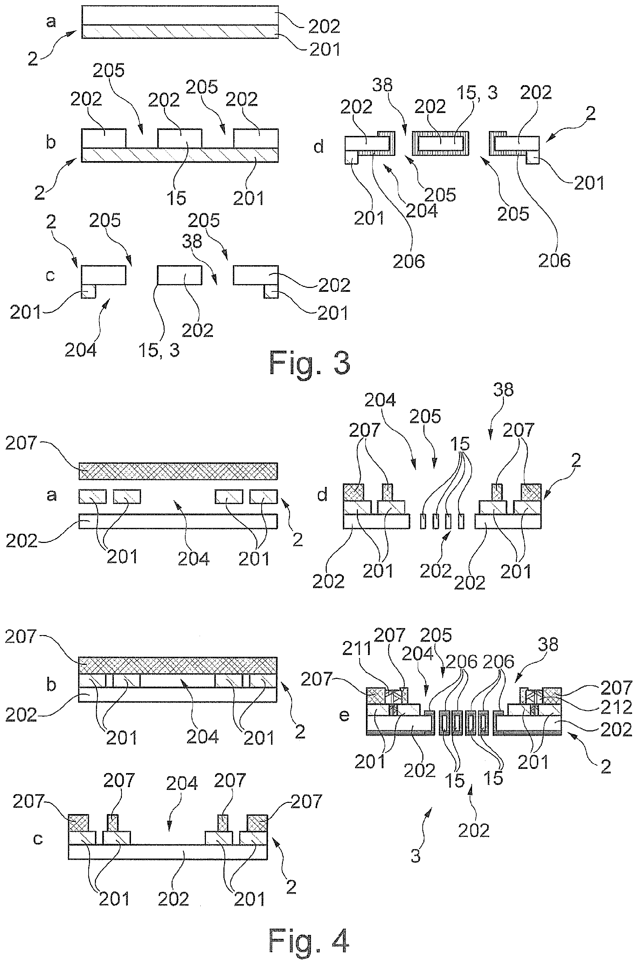

Das dem Verdampfer 3 zugeführte Liquid wird durch den Verdampfer 3 in Dampf/Aerosol umgesetzt. Der insbesondere elektrische Verdampfer 3 weist mindestens ein, vorzugsweise eine Mehrzahl von elektrischen Widerstands-Heizelementen 15 auf, siehe Figuren 3 und 4.

Die Verdampfervorrichtung 1 ist vorteilhaft in einer baulichen Einheit mit einem Flüssigkeitstank 6 als auswechselbare Kartusche 19 ausgeführt. Jede Kartusche 19 weist vorteilhaft ein Kartuschengehäuse auf, das mindestens teilweise von dem Flüssigkeitstank 6 und/oder mindestens teilweise von der Verdampfervorrichtung 1 gebildet sein kann. Der mindestens eine Flüssigkeitstank 6 kann wiederauffüllbar sein, so dass die Kartusche 19 ein wiederverwendbares Mehrwegteil ist. Die Kartusche 19 kann alternativ als Einwegteil ausgeführt sein.

Die Verdampfervorrichtung 1 bzw. jede Kartusche weist zweckmäßigerweise eine Dampfaustrittsöffnung auf, durch die erzeugter Dampf und/oder Aerosol aus der Verdampfervorrichtung 1 austreten und dem durch den Inhalator 27 fließenden Luftstrom beigemischt werden kann, um nach Austritt aus dem Inhalator 27 durch eine Zugöffnung von dem Konsumenten inhaliert zu werden.

Die Verdampfervorrichtung 1 umfasst vorteilhaft eine digitalelektronische Steuereinrichtung 4, beispielsweise eine anwendungsspezifische integrierte Schaltung (ASIC), die vorzugsweise auf dem Träger 2 angeordnet sein kann. Der oder die Verdampfer 3 der Verdampfervorrichtung 1 können durch die entsprechende elektronische Steuereinrichtung 4 individuell oder in Gruppen angesteuert und mit Strom aus einem Energiespeicher 46 beheizt werden, um an den Heizelementen 15 anliegende Flüssigkeit zu verdampfen.

Die Verdampfervorrichtung 1 bzw. die Kartusche 19 weist ein elektrisches Kontaktelement 7, hier in Form eines elektrischen Steckers, mit einer Mehrzahl von elektrischen Kontakten 10 auf. Die Kontakte 10 sind mittels elektrischer Leitungen mit der elektronischen Steuereinrichtung 4 verbunden, um Sensorsignale, Steuersignale und/oder elektrische Energie an das Basisteil 20 des Inhalators 27 zu übertragen. Die Verdampfervorrichtung 1 kann Sensoren, beispielsweise einen Temperatursensor zum Messen der Heiztemperatur und/oder einen Drucksensor zum Messen des Strömungsdrucks aufweisen.

Das Basisteil 20 umfasst eine Steuereinheit 29 und eine mit der Steuereinheit 29 verbundene oder zu verbindende Energiespeichereinheit 40. Die Steuereinheit 29 umfasst eine elektronische Steuerung 21 und ein mit dieser verbundenes elektrisches Kontaktelement 22, hier in Form einer zu dem Stecker 7 passenden Steckbuchse. Die Steuereinheit 29 umfasst vorteilhaft des Weiteren eine Nutzerschnittstelle 32, insbesondere eine Drahtlosschnittstelle, beispielsweise eine Bluetooth-Schnittstelle, über welche ein Nutzer den Inhalator 27 mittels eines mobilen Kommunikationsgeräts, beispielsweise eines Smartphones, steuern oder einstellen, und/oder Information von diesem erhalten kann. Die elektronische Steuerung 21 und das elektrische Kontaktelement 22 sind vorteilhaft auf einer gemeinsamen Leiterplatte 26 angeordnet. Die Gesamtheit aus elektronischer Steuereinrichtung 4 und elektronischer Steuerung 21 wird im Rahmen dieser Anmeldung als elektronische Steuervorrichtung 56 des Inhalators 27 bezeichnet.

Der elektrische Stecker 7 und die elektrische Steckbuchse 22 sind einander entsprechend eingerichtet, so dass durch Einstecken des Steckers 7 in die Steckbuchse 22 eine elektrische Verbindung zwischen der Verdampfervorrichtung 1 und dem Basisteil 20 zur Übertragung von Signalen, Daten und/oder elektrischer Leistung hergestellt wird. In der Verdampfervorrichtung 1 werden die Versorgungsströme und Signale von dem Stecker 7 an den mindestens einen Verdampfer 3 und/oder an Sensoren weitergeleitet. Vorteilhaft weisen der Stecker 7 und die Steckbuchse 22 jeweils die gleiche Anzahl von elektrischen Kontakten 10 auf.

Zum Verbinden der Verdampfervorrichtung 1 mit dem Basisteil 20 wird in manchen Ausführungsformen die Kartusche 19 parallel zur Längsachse des Basisteils 20 in dieses eingeschoben, wodurch der Stecker 7 in die Steckbuche 22 eingeschoben und die elektrische Verbindung hergestellt wird.

Vorteilhaft weist das Basisteil 20 bzw. die Steuereinheit 29 eine der Anzahl der Kartuschen 19 entsprechende Zahl von elektrischen Kontaktelementen 22, hier Steckbuchsen, auf, um den individuellen Austausch einzelner Kartuschen 19 zu ermöglichen.

In der elektronischen Steuereinrichtung 4 der Verdampfervorrichtung 1 ist vorteilhaft eine Kennung bzw. ID (Identifizierungsinformation) der Verdampfervorrichtung 1 dauerhaft gespeichert. Infolge des Verbindens der Kartusche 19 mit dem Basisteil 20 kann die elektronische Steuerung 21 die Kennung aus der Steuereinrichtung 4 auslesen und eine typgenaue und im Hinblick auf die jeweilige Flüssigkeit optimierte individuelle Steuerung des jeweiligen Verdampfers 3 und/oder der Verdampfervorrichtung 1 durchführen oder veranlassen, etwa durch Übermittlung von Steuer- und/oder Regelbefehlen an die Steuereinrichtung 4. In der elektronischen Steuerung 21 des Basisteils 20 sind zu diesem Zweck vorzugsweise Steuerdaten für eine Mehrzahl von Kennungen entsprechend einer Mehrzahl von unterschiedlichen Verdampfern 3 bzw. Verdampfertypen und/oder Flüssigkeiten gespeichert, beispielsweise in Form einer Datenbank.

Die Energiespeichereinheit 40 umfasst einen Energiespeicher 46, eine Batterieschnittstelle 41 zum Verbinden der Steuereinheit 29 mit der Energiespeichereinheit 40 über elektrische Leitungen 31, eine Ladeschnittstelle 42 und eine elektronische Schaltung mit Ladeelektronik. Die Steuereinheit 29 wird über die Batterieschnittstelle 41 mit Strom versorgt. Des Weiteren können über die Batterieschnittstelle 41 analoge und/oder digitale Signale zwischen der Energiespeichereinheit 40 und der Steuereinheit 29 übertragen werden. In einer vorteilhaften Ausführungsform umfassen die elektrischen Leitungen 31 einen digitalen Datenbus. Über die elektrische Verbindung 31 zwischen Basisteil 20 und Energiespeichereinheit 40 lassen sich beispielsweise Informationen über den Ladezustand des Energiespeichers 46 oder Diagnosedaten zwischen der Steuereinheit 29 und der Energiespeichereinheit 40 übermitteln. Der Energiespeicher 46 kann eine Einwegbatterie oder ein wiederaufladbarer Akku sein, beispielsweise ein Lithium-Ionen Akku, welcher über die Ladeschnittstelle 42, beispielsweise eine USB-Schnittstelle, oder vorteilhaft drahtlos über eine induktive Ladeschnittstelle, geladen werden kann.

Der oder jeder Flüssigkeitstank 6 bildet jeweils ein Flüssigkeitsreservoir 37A, 37B oder eine Mehrzahl von Flüssigkeitsreservoirs 37A, 37B aus. Insbesondere kann ein Mehrkammertank 6 zur Ausbildung mehrerer Reservoirs 37A, 37B, ... vorgesehen sein. Beispielhaft ist in Figur 1 eine Kartusche 19 mit einem Zweikammertank 6 zur Ausbildung zweier Reservoirs 37A, 37B vorgesehen. Selbstverständlich kann ein Flüssigkeitstank 6 auch mehr als zwei Reservoirs 37A, 37B ausbilden. In der Ausführungsform gemäß Figur 1 sind beispielhaft jeweils zwei Verdampfer 3 einem Reservoir 37A, 37B zugeordnet. Selbstverständlich können einem Reservoir 37A, 37B nur ein Verdampfer 3 oder mehr als zwei Verdampfer 3 zugeordnet sein.

Mischformen zwischen Ein- und Mehrkammertanks sind möglich, beispielsweise könnten ein Zweikammertank und ein Einkammertank vorgesehen sein.

Jedes Flüssigkeitsreservoir 37A, 37B, ... ist über eine zugeordnete Flüssigkeitszuführung 16 mit einem oder mehreren Verdampfern 3 verbunden, um Flüssigkeit von einer entsprechenden Öffnung des Flüssigkeitstanks 6 zu dem oder den Verdampfern 3 zu transportieren und dort zu verdampfen. Die Flüssigkeitszuführungen 16 können beispielsweise Durchgangsöffnungen 38 in einem Zwischenteil zwischen dem Träger 2 und dem Flüssigkeitstank 6 umfassen, siehe Figuren 3 und 4.

Zwischen jedem Verdampfer 3 und dem zugeordneten Flüssigkeitsreservoir 37A, 37B, ..., d.h. in der Flüssigkeitszuführung 16, ist vorteilhaft ein Kapillarelement 12 vorgesehen, das Flüssigkeit mittels Kapillarwirkung, beispielsweise mithilfe von Mikrokanälen, von dem Flüssigkeitstank 6 zu dem Verdampfer 3 fördert, um die Benetzung des Verdampfers 3 und die kontinuierliche Nachförderung von Liquid sicherzustellen. Das Kapillarelement 12 kann beispielsweise ein Porenelement mit optimierter Porengröße, ein offenporiges geschäumtes Element, ein Schwammelement und/oder eine Lamellenstruktur umfassen. Die Verdampfervorrichtung 1 und das kapillare Element 12 werden von einer Verdampfereinheit 90 umfasst.

Die eine oder mehrere Flüssigkeiten in den Reservoirs 37A, 37B, ... umfassen vorteilhaft eine oder mehrere Komponenten aus der Gruppe 1,2-Proplyenglykol, Glycerin, Wasser, mindestens einen Wirkstoff, insbesondere Nikotin, und/oder mindestens einen Aromastoff (Flavour) in gleichen und/oder unterschiedlichen Mischungsverhältnissen.

Die Verdampfervorrichtung 1 weist vorteilhaft eine standardisierte Flüssigkeitsschnittstelle 47 zur Anbindung des Flüssigkeitstanks 6 an die Verdampfervorrichtung 1, insbesondere deren Träger 2, auf. Die Flüssigkeitsschnittstelle 47 ist vorteilhaft an der dem Verdampfer 3 entgegengesetzten oder der dem Verdampfer zugewandten Seite des Trägers 2 angeordnet. An der Flüssigkeitsschnittstelle 47 wird das Liquid aus dem oder den Reservoirs 37A, 37B bereitgestellt und durch eine vorteilhafte Durchgangsöffnung durch den Träger 2 zu dem oder den Verdampfern 3 geleitet. Die Flüssigkeitsschnittstelle 47 kann beispielsweise mittels eines Dichtelements abgedichtet sein. Der Tank 6 kann für den Konsumenten lösbar oder unlösbar mit der Verdampfervorrichtung 1 verbunden sein. Die dicke und/oder Steifigkeit des Trägers kann vorzugsweise variieren und beispielsweise im Bereich der Heizelemente 15 dünn und flexibel sein, während die Anbindung an den Flüssigkeitstank 6 und/oder die äußeren der Halterung dienenden Ränder des Trägers 2 dick und/oder steif sind.

Figur 2 zeigt eine Ausführungsform des Trägers 2 der Verdampfervorrichtung 1 gemäß der Erfindung. Der Träger 2 ist folienbasiert und weist eine Polymerfolie 201 auf, welche vorzugsweise den Träger 2 seine Geometrie verleiht. An einer Kante 210 des Trägers 2 ist die Steckverbindung 7 ausgebildet, beispielsweise indem die Polymerfolie 201 dort mechanisch verstärkt, dicker und/oder biegesteifer als in anderen Bereichen des Trägers 2 ist. Der Träger 2 umfasst des Weiteren eine Metallfolie 202, welche Leiterbahnen und Kontakte 10, 211, 212, vorzugsweise auch die Kontakte 10 des Steckers 7, ausbildet. Der Verdampfer 3 erlaubt eine flächige Verdampfung von Liquid. Eine mögliche Realisierung eines flächigen Verdampfers mit separierbaren Bereichen ist in der Patentanmeldung DE 10 2016 120 803.5 beschrieben.

In dieser Ausführungsform weist der Träger 2 den Verdampfer 3 und die Steuereinrichtung 4 auf. Der Verdampfer 3 ist in den Träger integriert und die Heizelemente 15 des Verdampfers 3 werden durch die entsprechend strukturierte Metallfolie 202 ausgebildet. Die Steuereinrichtung 4 ist vorzugsweise mittels mehrerer Kontakte 211, 212, beispielsweise Lötkontakte oder Steckkontakte, mit der Verdampfervorrichtung 1 über Leiterbahnen elektrisch verbunden. Die Leiterbahnen befinden sich ebenfalls in der Metallfolie 202 und/oder in einer bevorzugten Ausführungsform in einer Metalllage 207, welche auch die Kontakte 10 ausbilden kann, siehe Figur 4.

Der Schaltungsträger 2 kann vorzugsweise direkt auf den Flüssigkeitstank 6 aufgebracht werden, wobei die fluidische Anbindung an den Flüssigkeitstank 6 direkt über eine Öffnung 38, 204, 205 unterhalb der Heizelemente 15 oder über die dazwischen angebrachte Kapillarstruktur 12 erfolgen kann. Die kapillare Struktur kann dabei vorzugsweise ein ein- oder mehrlagiges Vlies, beispielsweise aus Glasfasern, chemisch und thermisch stabilen Polymerfasern oder ähnlichem, sein. Der Träger 2 kann vorzugsweise auf eine flächige Öffnung des Flüssigkeitstanks 6 aufgebracht werden.

Durch den Zug eines Konsumenten am Inhalator 27 beaufschlagt der Konsument den Inhalator 27 mit einem Unterdruck, welcher einen Luftstrom impliziert. Der Luftstrom führt an der Verdampfervorrichtung 1, am Verdampfer 3 beziehungsweise den Heizelementen 15 vorbei und nimmt dort das durch den Verdampfer 3 generierte Aerosol beziehungsweise den Dampf mit und führt dieses beziehungsweise diesen den Konsumenten zu.

Figur 3 zeigt eine Ausführungsform eines Trägers 2 für eine erfindungsgemäße Verdampfervorrichtung 1 mit einem Schichtaufbau umfassend im Wesentlichen zwei Schichten 201, 202 und eine passivierende Beschichtung 206, welche ebenfalls als Schicht aufgefasst werden könnte. Die Figuren 3a bis 3d zeigen den Träger 2 in Schnittdarstellungen während verschiedener möglicher, aber nicht notwendiger Fertigungsschritte; die Fertigung der erfindungsgemäßen Verdampfervorrichtung 1 kann ebenso auf anderen als den beschriebenen Wegen geschehen.

Figur 3a zeigt den Träger 2 mit sich einander flächig kontaktierender Polymerfolie 201 und Metallfolie 202. Als Ausgangsmaterial für das Foliensubstrat der Polymerfolie 201 dient ein Polymer, welches nicht schmelzbar und chemisch beständig ist, beispielsweise Polyesterfolie oder Polyimide. Als Metallfolie 202 kann vorzugsweise eine auf die Polymerfolie 201 auflaminierte Kupferfolie 202 verwendet werden. Es können jedoch auch andere Metalle, wie zum Beispiel Nickel und andere Aufbringverfahren, wie zum Beispiel galvanische Abscheidung, Kleben und/oder Kaschieren, verwendet werden. Die Verbindung der Schichten des Trägers kann auch mittels Kleben erfolgen.

In Figur 3b erfolgte ausgehend von Figur 3a zunächst ein Fertigungsschritt, um die Metallfolie 202 zu bearbeiten und zu strukturieren. Die Metallfolie 202 kann vorzugsweise über Fotolithographie, Nassätzen und/oder mithilfe weiterer Verfahren strukturiert werden, um in vorzugsweise einem Fertigungsschritt sowohl Heizelemente 15 des Verdampfers 3 als auch Kontakte 10 für weitere elektronische Bauteile, wie die Steuereinrichtung 4 oder die Steckverbindung 7, als auch Leiterbahnen zu erzeugen. In anderen Fertigungsverfahren kann jedoch auch zuerst die Polymerfolie 201 bearbeitet werden.

Durch die Strukturierung der Metallfolie 202 bilden sich zweite Öffnungen 205. Die Bildung der zweiten Öffnungen 205 in der Metallfolie 202 bedeutet, dass die jeweils zweiten Öffnungen 205 durch verschiedene Bereiche der Metallfolie 202 begrenzt sind. Die verschiedenen Bereiche der Metallfolie 202, die die zweiten Öffnungen 205 begrenzen, können separat als elektrische Leiter dienen und somit Leiterbahnen und Heizelemente 15 ausbilden. In diesem Ausführungsbeispiel ist durch zwei zweite Öffnungen 205 ein dazwischen liegendes Heizelement 15 ausgebildet. In anderen Ausführungsformen, kann die Anzahl der zweiten Öffnungen 205 und der Heizelemente 15 variieren, siehe Figur 4.

Ein Resultat der Bearbeitung der Polymerfolie 201 ist in Figur 3c gezeigt. Die Polymerfolie 201 stellt eine erste Öffnung 204 bereit, welche beispielsweise über Plasmaätzen erzeugt werden kann. Die erste Öffnung 204 ist so angeordnet, dass sie mit der zweiten Öffnung 205 kommunizieren kann, d.h. dass Fluid durch den Träger 2 hindurchtreten kann, indem sowohl die erste Öffnung 204 als auch die zweite Öffnung 205 passiert wird. Die erste Öffnung 204 und die zweite Öffnung 205 bilden somit eine fluiddurchlässige Durchgangsöffnung 38. Der Transport von Flüssigkeit aus dem vorzugsweise unterhalb des Trägers 2 angeordneten Flüssigkeitstanks 6 zum Verdampfer 3 bzw. den Heizelementen 15 ist somit durch die Durchgangsöffnung 38 ermöglicht. Vorzugsweise ist die erste Öffnung 204 größer als die zweite Öffnung 205, um einen unnötigen Kontakt zwischen Heizelementen 15 und der Polymerfolie 201 sowie ein unnötiges Hindernis für den Flüssigkeitstransport durch die Durchgangsöffnung 38 zu vermeiden.

Nach einem vorzugsweise letzten und optionalen Fertigungsschritt ist, wie in Figur 3d gezeigt, eine passivierende Beschichtung 206 auf die Metalloberfläche der Metallfolie 202 im Bereich der Heizelemente 15 aufgebracht. Die passivierende Beschichtung 206 ist vorzugsweise dazu eingerichtet, freiliegende und die nicht die Polymerfolie 201 kontaktierenden Bereiche der Metallfolie 202 zu umschließen. Von der passivierenden Beschichtung 206 ausgenommen sind vorzugsweise dem Flüssigkeitstank 6 abgewandten Bereiche, welche zur Aufnahme von elektrischen Bauteilen, wie der Steuereinrichtung 4, dem Stecker 7, Kontakten 10, 211, 212 und/oder Leiterbahnen, dienen. Vorzugsweise sind all die Bereiche der Metallfolie 202 mit einer passivierenden Beschichtung 206 versehen, die potentiell in Kontakt mit dem Liquid aus dem Flüssigkeitstank 6 und/oder mit dem Dampf bzw. Aerosol kommen können. Dadurch verhindert die passivierende Beschichtung 206 den Transport von möglicherweise unerwünschten Stoffen, welche aus den Heizelementen 15 herausgelöst werden können.

Der folienbasierte Träger 2 ist durch den Schichtaufbau durch wenigstens die Polymerfolie 201 und die Metallfolie 202 wenigstens teilweise flexibel. Die Polymerfolie 201 und/oder die Metallfolie 202 können verschiedene Bereiche unterschiedlicher Dicke aufweisen. Dies kann genutzt werden, um die Biegesteifigkeit in den verschiedenen Bereichen unterschiedlich auszugestalten. Im Besonderen kann es von Vorteil sein, dass die Polymerfolie 201 am Rand des Trägers 2 besonders steif und/oder dick ist, um den Träger 2 eine bestimmte Form zu geben. Des Weiteren kann eine Kante 210 des Trägers 2 besonders steif und/oder dick ausgebildet sein, um zu ermöglichen die Steckverbinder 7 direkt in die Verdampfervorrichtung 1 zu integrieren. Ferner können stabilere und/oder dicker ausgebildete Bereiche des Trägers 2 dazu dienen, Kontakte 211, 212 bereitzustellen.

Die Bereitstellung von Liquid, welches an dem Heizelementen 15 verdampft wird, wird vorzugsweise durch die Flüssigkeitsschnittstelle 47 und/oder das kapillare Element 12 vermittelt. Die Flüssigkeitsschnittstelle 47 und/oder das kapillare Element 12 können sich dabei durch den Träger 2 durch die erste Öffnung 204 und die zweite Öffnung 205 bis zum Heizelement 15 beziehungsweise der Metallfolie 202 und/oder der passivierenden Beschichtung 206 erstrecken. Das kapillare Element 12 zusammen mit dem Träger 2 kann in bevorzugten Ausführungsformen als Verschluss des Flüssigkeitstanks 6 dienen.

Vorzugsweise ist die Verdampfervorrichtung 1 dazu eingerichtet, den Verdampfer 3 an den einzelnen, separat geregelten Heizelementen 15 bereichsweise anzusteuern beziehungsweise zu beheizen, um eine möglichst hohe Dampf- bzw. Aerosolqualität zu erzielen. Einzelne, separat geregelte Heizelemente 15 sind in der Patentanmeldung DE 10 2017 111 119.0 beschrieben.

Figur 4 zeigt eine weitere Ausführungsform eines Trägers 2 der Verdampfervorrichtung 1 mit einem Schichtaufbau umfassend im Wesentlichen drei Schichten 201, 202, 207 und die passivierende Beschichtung 206, welche ebenfalls als Schicht aufgefasst werden könnte. Die Figuren 4a bis 4e zeigen den Träger 2 in Schnittdarstellungen während verschiedener möglicher aber nicht notwendiger Fertigungsschritte; die Fertigung der erfindungsgemäßen Verdampfervorrichtung kann ebenso auf anderen als den beschriebenen Wegen geschehen.

Figur 4a zeigt die Bereitstellung der drei Schichten 201, 202, 207 des Trägers 2 umfassend die Polymerfolie 201 sowie die Metallfolie 202 und eine Metalllage 207. Die Metallfolie 202 ist auf der der Metalllage 207 gegenüberliegenden Seite der Polymerfolie 201 angebracht. Die Verwendung einer von der Metallfolie 202 separaten Metalllage 207 kann zur Erzielung einer optimalen Funktion erforderlich sein, da beispielsweise die Heizelemente 15 und elektrische Leiterbahnen, Kontakte 10, Steckverbinder 7 usw. aus unterschiedlichen Materialien gefertigt werden können. Dazu bietet sich ein wie in dieser Ausführungsform gezeigte im Wesentlichen dreilagiger Aufbau an. In dieser Ausführungsform kann es von Vorteil sein, wenn die Polymerfolie 201 bereits strukturiert wurde, beispielsweise durch Stanzen, Schneiden, Laserschneiden, Ätzen und/oder Plasmaätzen. In diesem Beispiel weist die Polymerfolie 201 eine erste Öffnung 204 sowie weitere Öffnungen für die Aufnahme von Kontakten 211, 212 auf.

Es kann von Vorteil sein, dass der Träger 2 unterschiedliche Bereiche verschiedener Dicken und/oder Steifigkeit aufweist, um der durch den Einbau in den Inhalator geforderten Geometrie gerecht zu werden.

Auf die strukturierte Polymerfolie 201 können wie in Figur 4b gezeigt auf der einen Seite der Polymerfolie 201 die Metalllage 207 aus einem gut leitenden Metall, wie beispielsweise Kupfer, und der anderen Seite der Polymerfolie 201 die Metallfolie 202, beispielsweise aus Nickel, Nickel-Eisen-Legierungen, oder ähnlichem aufgebracht, beispielsweise auflaminiert, worden sein.

Die Figuren 4c und 4d zeigen die Strukturierung der Metallfolie 202 und der Metalllage 207. In diesem Beispiel wird zuerst die Metalllage 207 und anschließend die Metallfolie 202 strukturiert. In anderen Ausführungsformen können diese Fertigungsschritte auch vertauscht oder zeitgleich vollzogen werden.

Die Metalllage 207 wird so strukturiert, dass sie vorzugsweise zusammen mit der Polymerfolie 201 dazu eingerichtet ist, Kontakte 211, 212 aufzunehmen und/oder Leiterbahnen und/oder Kontakte 10 auszubilden. Vorzugsweise bildet die Metalllage 207 die Kontakte 10 des Steckverbinders 7 der Verdampfervorrichtung 1 aus.

Durch die Strukturierung der Metallfolie 202 wird in dieser Ausführungsform eine Vielzahl von zweiten Öffnungen 205 ausgebildet. Jedes benachbarte Paar von zweiten Öffnungen 205 bildet einen als Heizelement 15 dienenden und separat ansteuerbaren elektrischen Widerstand aus. In diesem Ausführungsbeispiel bilden fünf zweite Öffnungen 205 vier Heizelemente 15 aus. Die Heizelemente 15 sowie die am Rand befindlichen Bereiche der Polymerfolie 201 begrenzen die zweiten Öffnungen 205. Die zweiten Öffnungen 205 sind so angeordnet, dass sie mit der ersten Öffnung 204 fluiddurchlässig kommunizieren und somit eine fluiddurchlässige Durchgangsöffnung 38 ausbilden.

In einem vorzugsweise letzten und optionalen Fertigungsschritt wird wie in Figur 4e gezeigt eine passivierende Beschichtung 206 auf die Metalloberfläche der Metallfolie 202 im Bereich der Heizelemente 15 aufgebracht. Die passivierende Beschichtung 206 ist vorzugsweise dazu eingerichtet die freiliegende und die nicht die Polymerfolie 201 und nicht die Metalllage 207 kontaktierende Metallfolie 202 zu umschließen, mit Ausnahme einzelner vorzugsweise dem Flüssigkeitstank 6 abgewandten Bereiche, welche zur Aufnahme von elektrischen Bauteilen wie der Steuereinrichtung 4, dem Stecker 7, Kontakten 10, 211, 212 und/oder Leiterbahnen dienen.

Vorzugsweise sind all die Bereiche der Metallfolie 202 mit einer passivierenden Beschichtung 206 versehen, die potentiell in Kontakt mit dem Liquid aus dem Flüssigkeitstank 6 und/oder mit dem Dampf bzw. Aerosol kommen können. Dadurch verhindert die passivierende Beschichtung 206 den Transport von möglicherweise unerwünschten Stoffen, welche aus den Heizelementen 15 herausgelöst werden können. Vorzugsweise nachdem die Metallfolie 202 strukturiert wurde, können auch die Kontakte 211, 212 durchkontaktiert werden, um zwischen der Metallfolie 201 und beispielsweise der Metalllage 207 und/oder in den Kontakten 211, 212 beispielsweise durch Lötzinn gehaltenen elektronischen Bauteilen, wie zum Beispiel der Steuereinrichtung 4, eine elektrische Verbindung herzustellen.

Bevorzugt wird die Metalllage 207 mit einer hier nicht dargestellten passivierenden Beschichtung 206 versehen, um den Transport von möglicherweise unerwünschten Stoffen, welche aus der Metalllage 207 herausgelöst werden können, zu verhindern. Die Eigenschaften der passivierenden Beschichtung 206 der Metalllage 207 können vorteilhaft denen der Metallfolie 202 entsprechen. Vorteilhaft dient die Metalllage 207 mit den Kontakten 211, 212 zur Erzeugung eines Schaltungsträgers.

In anderen nicht gezeigten Ausführungsformen kann die Anzahl der Öffnungen 204, 205 variieren.

Der mittlere Durchmesser beziehungsweise die Kantenlänge der zweiten Öffnungen 207 liegt vorzugsweise im Bereich zwischen 5 µm und 1 mm, weiter vorzugsweise im Bereich zwischen 30 µm und 250 µm, noch weiter vorzugsweise im Bereich zwischen 50 µm und 100 µm. Aufgrund dieser Abmessungen wird vorteilhaft eine Kapillarwirkung erzeugt, so dass vorzugsweise an der Flüssigkeitsschnittstelle 47 eindringende Flüssigkeit durch das kapillare Element 12 bis zum Verdampfer 3 nach oben steigt. Das Flächenverhältnis von zweiten Öffnungen 205 zu Heizelementen 15 liegt beispielsweise im Bereich zwischen 10% und 50%, vorteilhaft im Bereich zwischen 15% und 40%, noch weiter vorteilhaft im Bereich zwischen 20% und 30%, und beträgt beispielsweise 25%.

Die Kantenlängen der mit Heizelementen 15 versehenen Flächen der Verdampfervorrichtung 1 liegen beispielsweise im Bereich zwischen 0,5 mm und 3 mm. Die Abmessungen der mit Heizelementen 15 versehenen Flächen der Verdampfervorrichtung 1 können beispielsweise betragen: 0,95 mm x 1,75 mm; 1,9 mm x 1,75 mm oder 1,9 mm x 0,75 mm. Die Kantenlängen der Verdampfervorrichtung 1 beziehungsweise des Trägers 2 können beispielsweise im Bereich zwischen 0,5 mm und 5 mm liegen, vorzugsweise im Bereich zwischen 0,75 mm und 4 mm, weiter vorzugsweise im Bereich zwischen 1 mm und 3 mm liegen. Die Fläche der Verdampfervorrichtung 1 beziehungsweise des Trägers 2 (chip size) kann beispielsweise 1 mm x 3 mm oder 2 mm x 3 mm betragen.

Die Anzahl der zweiten Öffnungen 205 beziehungsweise der Heizelemente 15 liegt vorzugsweise im Bereich zwischen vier und 1000. Auf diese Weise lässt sich der Wärmeeintrag optimieren und eine gesicherte hohe Verdampfungsleistung sowie eine ausreichend große Dampfaustrittsfläche realisieren.

Die zweiten Öffnungen 205 sind vorteilhaft in Form eines quadratischen, rechteckigen, vieleckigen, runden, ovalen oder anders geformten Arrays angeordnet. Das Array kann in Form einer Matrix mit s Spalten und z Zeilen ausgebildet sein, wobei s vorteilhaft im Bereich zwischen 2 und 50 und weiter vorteilhaft im Bereich zwischen 3 und 30 und/oder z vorteilhaft im Bereich zwischen 2 und 50 und weiter vorteilhaft im Bereich zwischen 3 und 30 liegt. Auf diese Weise lässt sich eine effektive und auf einfache Weise herstellbare Anordnung der zweiten Öffnungen 205 beziehungsweise der Heizelemente 15 mit gesichert hoher Verdampfungsleistung realisieren.

Die Metallfolie 202 kann auch derart strukturiert sein, dass die sich durch die zweiten Öffnungen 205 ergebenden Formen der Heizelemente 15 oder die Former der zweiten Öffnungen 205 an bionische Strukturen anlehnen.

Der Querschnitt der zweiten Öffnungen 205 kann quadratisch, rechteckig, vieleckig, rund, oval oder anders geformt sein, und/oder sich in Längsrichtung abschnittweise ändern, insbesondere vergrößern, verkleinern oder konstant bleiben.

Die Mehrzahl von Heizelementen 15 kann bevorzugt unterschiedlich angesteuert beziehungsweise beheizt werden. Dabei kann die Dauer der einzelnen Verdampfungsschritte bei unterschiedlichen Temperaturen und/oder einem Verdampfen der einzelnen Komponenten der einzelnen Portionen der Flüssigkeit derart kurz gehalten werden und/oder mit einer Ansteuerfrequenz getaktet erfolgen, dass die schrittweise Verdampfung von einem Konsumenten nicht wahrgenommen und trotzdem eine weitgehend homogene, geschmackskonforme, wiederholbar präzise Aerosolbildung gewährleistet werden kann. Insbesondere erfolgt zunächst ein Verdampfen einer leichter siedenden Komponente der Flüssigkeit in einem ersten Verdampfungsintervall mit einer ersten Temperatur A und anschließend ein Verdampfen einer höher siedenden Komponente der Flüssigkeit in einem zweiten Verdampfungsintervall mit einer zweiten Temperatur B, welche die Temperatur A übersteigt.

Im Folgenden wird der Ablauf des Verdampfungsvorgangs erläutert.

Zum Verdampfen von Flüssigkeit wird eine Spannungsquelle für die Heizelemente 15 aktiviert. Die Spannung Uh wird dabei so eingestellt, dass die Verdampfungstemperatur in den Heizelementen 15 an das individuelle Verdampfungsverhalten des eingesetzten Flüssigkeitsgemischs angepasst ist. Dies verhindert die Gefahr von lokaler Überhitzung und dadurch Schadstoffentstehung.

Sobald eine Flüssigkeitsmenge verdampft ist, die dem Volumen der auf der Oberfläche des Verdampfers 3 befindlichen Flüssigkeit entspricht oder damit in Zusammenhang steht, wird die Heizspannungsquelle deaktiviert. Da die Liquideigenschaften und -menge vorteilhaft exakt bekannt sind und die Heizelemente 15 einen messbaren temperaturabhängigen Widerstand aufweisen, kann dieser Zeitpunkt sehr genau bestimmt bzw. gesteuert werden. Die Energieaufnahme der Verdampfervorrichtung 1 lässt sich daher gegenüber bekannten Vorrichtungen reduzieren, da die benötigte Verdampfungsenergie dosierter und damit exakter eingebracht werden kann.

Nach Abschluss des Heizvorgangs sind die Heizelemente 15 überwiegend oder vollständig von flüssigem Liquid befreit. Die Heizspannung wird dann so lange ausgeschaltet gehalten, bis mittels Nachförderung von Flüssigkeit durch beispielsweise das kapillare Element 12 die Oberfläche der Heizelemente 15 wieder benetzt ist. Sobald dies der Fall ist, kann der nächste Heizzyklus durch einschalten der Heizspannung begonnen werden.

Die von der Heizspannungsquelle erzeugte und von der Steuereinrichtung 4 geregelte Ansteuerfrequenz des Verdampfer 3 liegt vorteilhaft im Bereich von 1 Hz bis 50 kHz, bevorzugt im Bereich von 30 Hz bis 30 kHz, noch weiter vorteilhaft im Bereich von 100 Hz bis 25 kHz. Verschiedene Heizelemente 15 können mit unterschiedlichen, gleich, zeitlich variierenden oder zeitlich konstanten Ansteuerfrequenzen beheizt werden, um eine hohe und gleichbleibende Dampf- und/oder Aerosolqualität zu erzielen.

Die Frequenz und der Tastgrad der Heizspannung Uh für die Heizelemente 15 sind vorteilhaft an die Eigenschwingung bzw. Eigenfrequenz der Blasenschwingungen während der Blasensiedung angepasst. Vorteilhaft kann die Periodendauer 1/f der Heizspannung daher im Bereich zwischen 5 ms und 50 ms, weiter vorteilhaft zwischen 10 ms und 40 ms, noch weiter vorteilhaft zwischen 15 ms und 30 ms liegen und beispielsweise 20 ms betragen. Je nach Zusammensetzung der verdampften Flüssigkeit können andere als die genannten Frequenzen optimal an die Eigenschwingung bzw. Eigenfrequenz der Blasenschwingungen angepasst sein.

Des Weiteren hat sich gezeigt, dass der durch die Heizspannung Uh erzeugte maximale Heizstrom vorzugsweise nicht mehr als 7 A, weiter vorzugsweise nicht mehr als 6,5 A, noch weiter vorzugsweise nicht mehr als 6 A betragen und optimalerweise im Bereich zwischen 4 A und 6 A liegen sollte, um konzentrierten Dampf bei Vermeidung von Überhitzung zu gewährleisten.

Die Förderrate des kapillaren Elements 12 ist wiederum optimal an die an die Verdampfungsrate des Verdampfers 3 beziehungsweise der Heizelemente 15 angepasst, so dass jederzeit ausreichend Flüssigkeit nachgefördert werden kann.

## Patentansprüche

1. Verdampfervorrichtung (1) für einen Inhalator (27), insbesondere für ein elektronisches Zigarettenprodukt, umfassend
- einen elektrischen Verdampfer (3) mit einem oder mehreren Heizelementen (15) zum Verdampfen von dem Verdampfer (3) zugeführter Flüssigkeit, wobei
- ein Träger (2) aus einem Schichtsystem mit einer Polymerfolie (201) und mindestens einer die Polymerfolie (201) flächig kontaktierenden Metallfolie (202) gebildet ist,
- die Polymerfolie (201) wenigstens eine fluiddurchlässige erste Öffnung (204) aufweist,
- die Metallfolie (202) wenigstens eine mit der ersten Öffnung (204) kommunizierende fluiddurchlässige zweite Öffnung (205) aufweist, und
- die Metallfolie (202) die Heizelemente (15) ausbildet, wobei die Heizelemente (15) die zweite Öffnung (205) begrenzend angeordnet sind, **dadurch gekennzeichnet, dass**
- der Träger (2) eine Metalllage (207) umfasst und die Metalllage (207) die Polymerfolie (201) auf der der Metallfolie (202) gegenüberliegenden Seite kontaktiert.

2. Verdampfervorrichtung nach Anspruch 1, **dadurch gekennzeichnet, dass**
- die Metallfolie (202) wenigstens an den die Polymerfolie (201) nicht kontaktierenden Abschnitten und/oder den Heizelementen (15) eine passivierende Beschichtung (206) aufweist.

3. Verdampfervorrichtung nach einem der vorangehenden Ansprüche, **dadurch gekennzeichnet, dass**
- der Träger (2) wenigstens einen Kontakt (211, 212) zum elektrischen Kontaktieren eines elektronischen Bauteils (4, 56) aufweist.

4. Verdampfervorrichtung nach einem der vorangehenden Ansprüche, **dadurch gekennzeichnet, dass**
- der Träger (2) Bereiche unterschiedlicher Dicke und/oder Biegesteifigkeit aufweist.

5. Verdampfervorrichtung nach einem der vorangehenden Ansprüche, **dadurch gekennzeichnet, dass**
- der Träger (2) eine Flüssigkeitsschnittstelle (47) für eine Verbindung mit einem Flüssigkeitstank (6) aufweist, wobei die Flüssigkeitsschnittstelle (47) mit der ersten Öffnung (204) und/oder der zweiten Öffnung (205) kommuniziert.

6. Verdampfervorrichtung nach einem der vorangehenden Ansprüche, **dadurch gekennzeichnet, dass**
- an einer Kante (210) des Trägers (2) ein elektrisches Steckverbinderteil (7) ausgebildet ist, das zum reversiblen Zusammenwirken mit einem entsprechenden Steckverbinderteil (22) eines Basisteils (20) des Inhalators (27) und zur Versorgung der Verdampfervorrichtung (1) mit elektrischer Energie und/oder zum Empfangen von Steuersignalen für die Verdampfervorrichtung (1) eingerichtet ist.

7. Verdampfervorrichtung nach einem der vorangehenden Ansprüche, **dadurch gekennzeichnet, dass**
- eine elektronische Steuereinrichtung (4) zum Steuern und/oder Regeln der Verdampfervorrichtung (1) auf dem Träger (2) gehalten ist.

8. Verdampfereinheit (90) für einen Inhalator (27) umfassend
- eine Verdampfervorrichtung (1) nach einem der vorangehenden Ansprüche, und
- ein kapillares Element (12) zur Förderung von Fluid zu dem Verdampfer (3).

9. Verdampfereinheit nach Anspruch 8, **dadurch gekennzeichnet, dass** sich das kapillare Element (12) wenigstens teilweise durch die erste Öffnungen (204) und/oder durch die zweite Öffnung (205) in dem Träger (2) erstreckt.

10. Kartusche (19) für einen Inhalator (27), umfassend
- wenigstens eine Verdampfervorrichtung (1) oder Verdampfereinheit (90) nach einem der vorangehenden Ansprüche, und
- einen Flüssigkeitstank (6).

11. Inhalator (27) umfassend wenigstens eine Verdampfervorrichtung (1), Verdampfereinheit (90) oder Kartusche (19) nach einem der vorangehenden Ansprüche und ein damit kommunizierendes Basisteil (20).

12. Verfahren zur Fertigung einer Verdampfervorrichtung (1) nach einem der Ansprüche 1 bis 7, **dadurch gekennzeichnet, dass** der Träger (2) durch folgende Schritte aufgebaut wird:
- die Polymerfolie (201) und die Metallfolie (202) werden flächig miteinander verbunden,
- die Metallfolie (202) wird strukturiert, um die zweite Öffnung (204), Leiterbahnen und/oder Heizelemente (15) auszubilden,
- die Polymerfolie (201) wird strukturiert, um die erste Öffnung (203) auszubilden.

13. Verfahren nach Anspruch 12, **dadurch gekennzeichnet, dass**
- die Metallfolie (202) mit einer passivierenden Beschichtung (206) versehen wird.

14. Verfahren nach Anspruch 12 oder 13, **dadurch gekennzeichnet, dass**
- eine Metalllage (207) auf der der Metallfolie (202) gegenüberliegenden Seite der Polymerfolie (201) mit der Polymerfolie (201) verbunden wird.

15. Verfahren nach Anspruch 14, **dadurch gekennzeichnet, dass**
- die Metalllage (207) mit einer passivierenden Beschichtung (206) versehen wird.

## Claims

1. Vaporizer device (1) for an inhaler (27), in particular for an electronic cigarette product, comprising:
- an electrical vaporizer (3) having one or more heating elements (15) for vaporizing liquid supplied to the vaporizer (3),
wherein
- a carrier (2) is formed of a layer system with a polymer film (201) and at least one metal film (202) in contact with the polymer film (201) in a planar manner,
- the polymer film (201) comprises at least one fluid permeable first opening (204),
- the metal film (202) comprises at least one fluid permeable second opening (205) communicating with the first opening (204), and
- the metal film (202) forms the heating elements (15), wherein the heating elements (15) are arranged delimiting the second opening (205), **characterized in that**
- the carrier (2) comprises a metal layer (207), and the metal layer (207) is in contact with the polymer film (201) on the side opposite from the metal film (202).

2. Vaporizer device according to claim 1, **characterized in that**
- the metal film (202) has a passivating coating (206) at least on the sections not in contact with the polymer film (201) and/or the heating elements (15).

3. Vaporizer device according to any one of the preceding claims, **characterized in that**
- the carrier (2) comprises at least one contact (211, 212) for making electrical contact with an electronic component (4, 56).

4. Vaporizer device according to any one of preceding claims, **characterized in that**
- the carrier (2) comprises regions having different thicknesses and/or bending strengths.

5. Vaporizer device according to any one of the preceding claims, **characterized in that**
- the carrier (2) comprises a liquid interface (47) for a connection with a liquid tank (6), wherein the liquid interface (47) communicates with the first opening (204) and/or the second opening (205).

6. Vaporizer device according to any one of the preceding claims, **characterized in that**
- an electrical plug connector part (7) is formed on an edge (210) of the carrier (2), which is configured for reversible interaction with a corresponding plug connector part (22) of a base part (20) of the inhaler (27) and for supplying the vaporizer device (1) with electrical energy and/or for receiving control signals for the vaporizer device (1).

7. Vaporizer device according to any one of the preceding claims, **characterized in that**
- an electronic control device (4) for controlling and/or regulating the vaporizer device (1) is mounted on the carrier (2).

8. Vaporizer unit (90) for an inhaler (27) comprising:
- a vaporizer device (1) according to any one of the preceding claims, and
- a capillary element (12) for conveying fluid to the vaporizer (3).

9. Vaporizer unit according to claim 8, **characterized in that** the capillary element (12) extends at least partially through the first openings (204) and/or through the second opening (205) in the carrier (2).

10. Cartridge (19) for an inhaler (27), comprising:
- at least one vaporizer device (1) or vaporizer unit (90) according to any one of the preceding claims, and
- a liquid tank (6).

11. Inhaler (27) comprising at least one vaporizer device (1), vaporizer unit (90) or cartridge (19) according to any one of the preceding claims and a base part (20) communicating therewith.

12. Method for fabricating a vaporizer device (1) according to any one of claims 1 to 7, **characterized in that** the carrier (2) is made by way of the following steps:
- the polymer film (201) and the metal film (202) are connected to each other in a planar manner,
- the metal film (202) is structured in order to form the second opening (204), conducting tracks and/or heating elements (15),
- the polymer film (201) is structured in order to form the first opening (203).

13. Method according to claim 12, **characterized in that**
- the metal film (202) is provided with a passivating coating (206).

14. Method according to claim 12 or 13, **characterized in that**
- a metal layer (207) is connected to the polymer film (201) on the side of the polymer film (201) that is opposite from the metal film (202).

15. Method according to claim 14, **characterized in that**
- the metal layer (207) is provided with a passivating coating (206).

## Revendications

1. Dispositif évaporateur (1) pour un inhalateur (27), en particulier pour un produit de cigarette électronique, comprenant
- un évaporateur (3) électrique doté d'un ou de plusieurs éléments chauffants (15) pour l'évaporation de liquide introduit dans l'évaporateur (3), dans lequel
- un support (2) est formé à partir d'un système de couches avec une feuille de polymère (201) et au moins une feuille de métal (202) qui vient en contact par sa surface avec la feuille de polymère (201),
- la feuille de polymère (201) comporte au moins une première ouverture (204) perméable aux fluides,
- la feuille de métal (202) comporte au moins une seconde ouverture (205) perméable aux fluides communiquant avec la première ouverture (204) et
- la feuille de métal (202) forme les éléments chauffants (15), les éléments chauffants (15) étant disposés de manière délimitant la seconde ouverture (205), **caractérisé en ce que**
- le support (2) comprend une couche de métal (207) et la couche de métal (207) vient en contact avec la feuille de polymère (201) sur le côté opposé à la feuille de métal (202).

2. Dispositif évaporateur selon la revendication 1, **caractérisé en ce que**
- la feuille de métal (202) comporte un revêtement de passivation (206) au moins sur les parties qui ne viennent pas en contact avec la feuille de polymère (201) et/ou sur les éléments chauffants (15).

3. Dispositif évaporateur selon l'une des revendications précédentes, **caractérisé en ce que**
- le support (2) comporte au moins un contact (211, 212) pour la mise en contact électrique d'un composant électronique (4, 56).

4. Dispositif évaporateur selon l'une des revendications précédentes, **caractérisé en ce que**
- le support (2) comporte des zones avec une épaisseur et/ou une rigidité à la flexion différentes.

5. Dispositif évaporateur selon l'une des revendications précédentes, **caractérisé en ce que**
- le support (2) comporte une interface pour liquides (47) pour établir une liaison avec un réservoir de liquide (6), l'interface pour liquides (47) communiquant avec la première ouverture (204) et/ou la seconde ouverture (205).

6. Dispositif évaporateur selon l'une des revendications précédentes, **caractérisé en ce que**
- sur un bord (210) du support (2), un élément connecteur électrique (7) est formé, qui est configuré pour la coopération réversible avec un élément connecteur (22) correspondant d'une partie de base (20) de l'inhalateur (27) et pour l'alimentation du dispositif évaporateur (1) en énergie électrique et/ou pour la réception de signaux de commande pour le dispositif évaporateur (1).

7. Dispositif évaporateur selon l'une des revendications précédentes, **caractérisé en ce que**
- un moyen de commande (4) électronique pour commander et/ou réguler le dispositif évaporateur (1) est maintenu sur le support (2).

8. Unité évaporateur (90) pour un inhalateur (27) comprenant
- un dispositif évaporateur (1) selon l'une des revendications précédentes, et
- un élément capillaire (12) pour le transport de fluide vers l'évaporateur (3).

9. Unité évaporateur selon la revendication 8, **caractérisée en ce que** l'élément capillaire (12) s'étend dans le support (2) au moins en partie à travers la première ouverture (204) et/ou à travers la seconde ouverture (205).

10. Cartouche (19) pour un inhalateur (27), comprenant
- au moins un dispositif évaporateur (1) ou une unité évaporateur (90) selon l'une des revendications précédentes, et
- un réservoir de liquide (6).

11. Inhalateur (27) comprenant au moins un dispositif évaporateur (1), une unité évaporateur (90) ou une cartouche (19) selon l'une des revendications précédentes et une partie de base (20) communiquant avec celui-ci ou celle-ci.

12. Procédé de fabrication d'un dispositif évaporateur (1) selon l'une des revendications 1 à 7, **caractérisé en ce que** le support (2) est construit au moyen des étapes suivantes :
- la feuille de polymère (201) et la feuille de métal (202) sont reliées l'une à l'autre par leur surface,
- la feuille de métal (202) est structurée pour former la seconde ouverture (204), des pistes conductrices et/ou des éléments chauffants (15),
- la feuille de polymère (201) est structurée pour former la première ouverture (203).

13. Procédé selon la revendication 12, **caractérisé en ce que**
- la feuille de métal (202) est pourvue d'un revêtement de passivation (206).

14. Procédé selon la revendication 12 ou 13, **caractérisé en ce que**
- une couche de métal (207) est reliée à la feuille de polymère (201) sur le côté de la feuille de polymère (201) opposé à la feuille de métal (202).

15. Procédé selon la revendication 14, **caractérisé en ce que**
- la couche de métal (207) est pourvue d'un revêtement de passivation (206).
